# EUROPEAN PATENT SPECIFICATION

(11) **EP 2 818 539 B1**
(45) Date of publication and mention of the grant of the patent: **02.08.2017**
(21) Application number: 14174065.4
(22) Date of filing: 26.06.2014
(51) Int. Cl.: C12G 1/02, C12M 1/36, G01N 33/14

(54) **Apparatus for monitoring and controlling fermentation processes**
Vorrichtung zur Überwachung und Kontrolle von Fermentationsprozessen
Appareil pour surveiller et réguler des procédés de fermentation

(30) Priority: 28.06.2013 IT VR20130152
(43) Date of publication of application: 31.12.2014
(73) Proprietor: Innotec - Tecnologie Innovative S.r.l., 37135 Verona (IT)
(72) Inventor: Zavarise, Luca, 37010 Costermano, Fraz. Marciaga VR (IT)
(74) Representative: Modiano, Micaela Nadia

(56) References cited:
- DE-A1-102007 027 914
- US-A1- 2003 097 937
- Claire Swedberg: "UC Davis Winery Tracks Fermentation Via RFID Sensors", RFID Journal, 24 November 2010 (2010-11-24), pages 1-2, XP055083555, Hauppauge, NY Retrieved from the Internet: URL:http://www.rfidjournal.com/articles/vi ew?8033/ [retrieved on 2013-10-11]
- Marijan Culina ET AL: "GEA Diessel GmbH uses intelligent HAMILTON ARC sensors for pH-, DO and conductivity measurement in a process fermentation plant", , November 2010 (2010-11), pages 1-2, XP055083590, Hildesheim, Germany Retrieved from the Internet: URL:http://www.hamiltoncompany.com/downloa ds/695099R00%20AN%20ARC%20in%20GEA%20ferme ntation%20plants%20en.pdf [retrieved on 2013-10-11]
- "PORTABLE ANALYSIS SYSTEM FOR CONTROLLING FERMENTATION", , November 2012 (2012-11), pages 1-2, XP055083564, Parma, Italy Retrieved from the Internet: URL:http://www.maselli.com/wp-content/uplo ads/2013/09/if02-2931gb.pdf [retrieved on 2013-10-11]
- "FERMENTATION MONITOR IF02 (Receiver FM01 with RP60)", , August 2012 (2012-08), pages 1-2, XP055083588, Parma, Italy Retrieved from the Internet: URL:http://www.maselli.com/wp-content/uplo ads/2013/09/if02-1340gb1.pdf [retrieved on 2013-10-11]
- Claire Swedberg: "Boutique Winery Believes RFID Offers a Barrelful of Benefits", RFID Journal, 15 December 2008 (2008-12-15), pages 1-2, XP055083561, Hauppauge, NY Retrieved from the Internet: URL:http://www.rfidjournal.com/articles/vi ew?4514 [retrieved on 2013-10-11]
- "Arc View Handheld Package Specification Sheet", , 30 May 2012 (2012-05-30), XP055083595, USA Retrieved from the Internet: URL:http://www.hamiltoncompany.com/product s/sensors/c/876/ [retrieved on 2013-10-11]

## Description

The invention relates to an apparatus for monitoring and controlling fermentation processes.

As is known, in the food industry and, more specifically, in the wine industry, production is based on the fermentation of sugars contained in musts.

The biological, chemical and physical processes that go to make up the conversion of musts into wines are very complex, mutually interacting and highly dependent on the environmental conditions in which they take place. Managing such processes is an index of quality of the expected end result.

The technology available today makes it possible to monitor fermentations by way of laboratory analyses conducted on samples taken from the tanks in which the fermenting product is contained.

After an assessment by technologists of the results obtained from the analyses, consequent intervention actions are adopted.

The analytical data, together with the notes and with the actions adopted, are generally filed in hardcopy form, such as department journals, or are partially filed in computer files associated with management software packages.

Evidently this way of operating involves an extremely high use of specialist personnel in order to take the samples directly from the tank and at the various depths.

Furthermore, it requires immediate analysis, in order to prevent the conditions of the sample from differing from the evolving product inside the tank from which it was taken.

In reality, this rapidity of analysis is not feasible. The time that elapses between taking the samples and analyzing them is always lengthy and, therefore, the analytical results must be interpreted by technologists and always used as a delayed reference with respect to the actual state of the fermentation underway.

Likewise, also available are several types of sensor that can be installed, which are fixed to the tanks used for the conversion processes.

The data detected by such sensors can be transmitted using various different data transmission methods, typically networks or field buses or even via wireless connections, to a control unit.

The installation of analysis sensors fixed directly to the fermentation tanks makes it possible to obtain a continuous flow of analytical results and makes it possible to store such results in a database or to send them to a data processing system, which as of the present time is however only partially developed.

The limitation of this approach is due to the enormous deployment of economic resources that is necessary in order to completely equip the tanks used in the fermentation processes with the analysis sensors.

Another aspect to consider is that, currently, the procedure for managing the processes of conversion of the product are contained, usually, in protocols written by technologists, but the daily actions and practical instructions to the operators are given orally or with forms filled in. Such forms, once the instructions contained in them have been executed, are usually filed. The verification of the work carried out depends, therefore, on the operator's confirmation alone and on a possible laboratory analysis. Traceability is ensured only if procedures exist, and if they are correctly followed. Evidently, during the busiest season, such procedures are considered bureaucracy of secondary importance, since the operators do not have the necessary time to carry them out.

In order for the fermentation process to take place correctly, control of the temperature of the must is also important.

In particular, thermal conditioning of the fermenting musts is usually done with the use of forced circulation of cooling or heating solutions within heat exchangers, which, dynamically or statically, exchange heat with the fermenting product.

Thermal conditioning thus described is largely widespread and, when present, it is carried out automatically. To this end, each tank present in the cellar is provided with a sensor that continuously reads the temperature, so that it can be maintained at a constant preset value.

The temperatures read by the sensor are generally gathered in a station, which makes it possible for the operator and technologists to monitor and intervene as necessary.

When management is done with a PLC-and-PC system or a PC-only system, it is also possible to process the data, generating statistics on the trend of the temperature.

However, the technical solution adopted nowadays is geared only to ensuring that a constant temperature value of the fermenting product is maintained, which is completely lacking an approach that makes it possible to process the measured data in order to find a correlation between the action of thermal conditioning and the fermentation proper which generates a request for cooling or heating of the must. Furthermore, the current solution does not document the state of quiescent fermentation or when fermentation has stopped, since it does not track the lack of a thermal conditioning request.

As is likewise known, the fermentation process generates high quantities of carbon dioxide and the production of carbon dioxide is directly proportional to the stage to which the fermentation has advanced. Solutions exist which are based on measuring the quantity of carbon dioxide that exits from the fermentation tanks, in order to indirectly monitor the trend of fermentation.

A limitation of this type of solution derives from the high solubility of carbon dioxide at different temperatures, which greatly affects the consequent evaluations.

Furthermore, the detection of carbon dioxide makes it necessary to keep the tanks closed during fermentation, in order to operate under a slight overpressure and thus be able to channel the carbon dioxide produced by the fermentation process toward measurement systems. This, however, determines a considerable complication in terms of construction.

Other solutions that involve monitoring the fermentation by way of indirect measurement systems use sensors that measure the deviation of weight or, in other words, of density of the must during the fermentation. In fact, the specific weights of the musts decrease significantly during the fermentation process.

Such solutions use, in essence, pressure sensors, which are arranged on the bottom of the tank, so as to weigh the column of liquid that rests on them and, on the basis of the measurements taken, an adapted software program can be used to extrapolate the current fermentation states.

However, the results that can thus be obtained can easily be altered by various physical phenomena, such as the sedimentation of turbid particles, the variation of the temperature and, thus, the release or the solubilization of carbon dioxide, and the changes of level.

Furthermore, this is still an indirect measurement of the state of the fermentation. C. Swedeberg, "UC Davis Winery Tracks Fermentation Via RFID Sensors", RFID Journal, 24 November 2010 (http://www.rfidjournal.com/articles/ view?8033/), discloses a survey system for a winery having sensors for temperature and sugar content in each fermentation tank which each have their own unique RFID tag which can communicate wirelessly with a hub (laptop), the hub being in wireless communication with a central data processing system on Internet server. The system allows for automated analysis of the fermentation process and alerts the user if the sensor readings are outside a specified range.

M Culina et al., "GEA Diessel GmbH uses intelligent HAMILTON ARC sensors for pH-, DO and conductivity measurement in a process fermentation plant", November 2010 (http://www.hamiltoncompany.com/downloads/695099R00%20AN%20ARC %20in%20GEA%20fermentation %20plants%20en.pdf), discloses a survey system for fermentation using sensors (e.g. pH, conductivity, oxygen) with wireless interfaces distributed in the process plant, e.g. inside fermentors, having portable control unit in wireless communication with the sensors as well as connection to a production control system.

A portable measurement probe for samples taken from a fermentation process is known from Maselli Misure, "Portable Analysis System for Controlling Fermentation", November 2012 (http://www.maselli.com/wp-content/ uploads/2013/09/if02-2931gb.pdf). It can perform measurements at fermentation tanks and transfer the data to a central unit.

The aim of the present invention is to provide an apparatus for monitoring fermentation processes that is capable of solving the drawbacks of the known art, by making it possible to directly measure the fermentation process in relatively short times and at low cost.

Within this aim, an object of the present invention is to provide an apparatus for monitoring fermentation processes that makes it possible to perform a high number of analyses on the fermenting product even in the course of the same day, without requiring high plant and operating costs.

Another object of the invention is to provide an apparatus for monitoring fermentation processes that makes it possible to directly measure the chemical/physical parameters involved in the fermentation.

Another object of the invention is to provide an apparatus that makes it possible to perform analyses on the fermenting product in short times, so as to be able to take prompt corrective intervention action.

Another object of the present invention is to make available an apparatus for monitoring fermentation processes that enables technologists to very simply and immediately interpret the results of the analyses, so as to facilitate them in consideration of their scant availability of time.

This aim and these and other objects which will become better apparent hereinafter are all achieved by an apparatus for monitoring fermentation processes, according to the invention, as defined in appended claim 1.

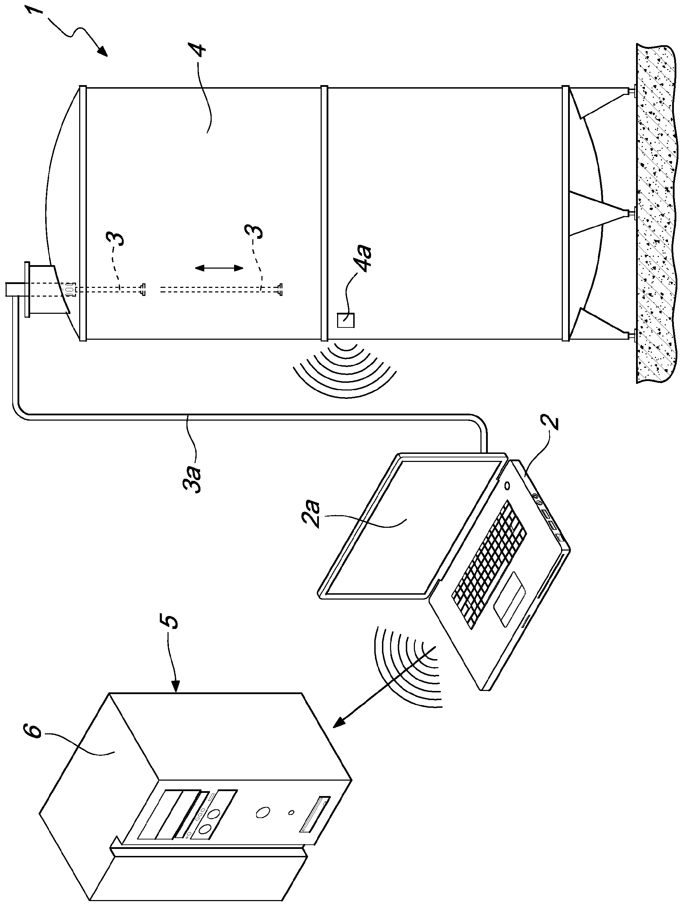

Further characteristics and advantages of the invention will become better apparent from the detailed description that follows of a preferred, but not exclusive, embodiment of the apparatus, according to the invention, which is illustrated by way of non-limiting example in the accompanying drawings wherein Figure 1 schematically illustrates the apparatus according to the invention.

With reference to the sole figure, the apparatus for monitoring fermentation processes, according to the invention, which is generally designated with the reference numeral 1, comprises a portable control unit 2, which is provided with means for communication with a detection probe 3, which can be immersed at different height levels within a fermenting product contained in a tank 4 that is installed, for example, in a cellar for the production of alcoholic beverages, such as wine, beer or the like.

In particular, the probe 3 is provided with at least one sensor that is capable of reading a respective parameter that is correlated directly with the fermentation process that is underway in the product present in the tank 4.

The portable control unit 2 is, likewise, provided with means for treating the data that originate from the probe 3, so as to enable the user, who might be, for example, an ordinary cellar operator, to perform the appropriate evaluations of the state of the fermentation underway in the product contained in the tank 4, as will be better explained hereinbelow.

Conveniently, the communication means between the probe 3 and the portable control unit 2 can be constituted by a data transmission cable 3a. In any case, there is no reason why such communication means cannot also be of the wireless type.

In order to enable the use of the apparatus according to the invention in cellars with several different tanks 4, the portable control unit 2 is, advantageously, provided with recognition means of each individual tank 4 that is present in the cellar.

Such recognition means comprise at least one unique code that is associated with the individual tank 4 and means of reading such unique code, which are associated with the portable control unit 2.

In essence, the unique code can, for example, be constituted by a radio signal emitted by an RFID electronic circuit 4a, fixed on the tank 4 and, in such case, the means of reading are provided by a receiver of the radio signal emitted by the RFID electronic circuit 4a.

Conveniently, the means for treating the data that originate from the probe 3 comprise a memory, which is loaded with a preset work protocol developed by the technologist on the basis of the desired trend of the fermentation, and by processing means that make it possible to compare the data received from the probe 3 with the work protocol, so as to enable the user or the technologist to detect any deviations of the fermentation underway with respect to the work protocol.

In particular, the work protocol can be set by the technologist in the period before the start of fermentation and can be set by adapting it to the vintage and to the production planning of the winery. Furthermore, in the work protocol, the technologist can plan any intervention actions in response to the appearance of phenomena of deviation of the fermentation parameters with respect to the set protocol.

Advantageously, the portable control unit 2 comprises at least one visual interface 2a, which is constituted, for example, by a monitor or the like, is connected to the processing means, and makes it possible to display, for example, the data originating from the probe 3.

Conveniently, the portable control unit 2 is also associated with an interface for entering data, which is constituted, for example, by a keyboard or by a reader of memory devices such as USB flash drives, CD-ROMs or the like, which enables the user to load the work protocols or information about the fermentation process to be monitored.

By way of example, the portable control unit 2 can consist of a computer of the portable type, optionally provided with an adapted accommodation case, in order to enable an easy and safe transport thereof within the cellar.

Conveniently, the probe 3 is provided with at least one level sensor the function of which is to tell the portable control unit 2 the position reached by the probe inside the tank 4.

Conveniently, by way of the visual interface 2a the portable control unit 2 is capable of emitting a first signal upon reaching, on the part of the probe 3, at least one preset level of height from the bottom of the tank 4, so as to let the user know when to stop the probe 3 inside the tank 4 in order to begin reading the parameters that are directly correlated with the fermentation underway.

Conveniently, the visual interface 2a is also adapted to emit at least one second signal, for example upon reaching a preset period of time, on the part of the probe 3, at the above mentioned preset height level, so as to alert the user when the reading operations by the probe 3 are concluded.

Advantageously, the portable control unit 2 is, furthermore, provided with wireless transceiver means of communicating information to a second control unit 5.

Such second control unit 5 can comprise a PC or a smartphone, which can be used by the technologist tasked with overseeing the fermentation processes in the cellar, such as, for example, the enologist responsible for the production, in order to enable him/her to obtain the data that the portable control unit 2 receives from the probe 3.

Conveniently, the second control unit 5 can comprise a fixed control station 6, which is installed, conveniently, in the cellar, and is provided with a data storage database, into which is entered all the data read by the probe 3 in the various tanks of the cellar and, optionally, processed by the portable control unit 2.

Advantageously, the processing means associated with the portable control unit 2 are adapted to send, by way of the above mentioned wireless transceiver means, an intervention signal to the second control unit 5, in the event of detection, by the processing means, of a deviation between the data collected by the probe 3 and the work protocol loaded in the memory of the portable control unit 2.

For example, such intervention signal can consist of an SMS text message, if the second control unit 5 is constituted by a smartphone, or it can consist of an email or other type of message that can be received by the second control unit 5, if the latter is constituted by a PC.

In this manner, the technologist can be alerted promptly when some parameters read by the probe 3 do not conform to the desired values specified in the work protocol, thus enabling him/her to promptly adopt the necessary corrective measures.

Conveniently, the probe 3 has a substantially tubular body and can comprise, in addition to the level sensor, a plurality of sensors of different types.

Preferably, the probe 3 can comprise one or more sensors selected, for example, from among: an oxygen sensor, a conductivity sensor, an ammonia nitrogen sensor, a turbidity sensor, a pH sensor, a sugar sensor, a temperature sensor or other type of sensor adapted to the monitoring of the fermenting process underway in the product contained in the tank 4.

Advantageously, the portable control unit 2 is also provided with means for receiving the data that arrive from means for measuring the heat exchanged between the tank 4 and a heat exchanger that is associated with the tank 4 and designed to control the temperature of the fermenting product.

The data obtained by way of the above mentioned means of measuring the exchanged heat can be represented using the visual interface 2a of the portable control unit 2 in order to enable the user to check the conditions of the fermentation underway in the product contained in the tank 4.

Conveniently, such measurement means comprise at least one flow meter that is adapted to read the flow-rate of the heat exchange liquid that flows through the heat exchanger associated with the tank 4, and temperature sensors, which are designed to read the entry temperature and the exit temperature of the heat exchange fluid into and out of the heat exchanger.

Operation of the apparatus according to the invention is the following.

The user brings the portable control unit 2 in proximity to the tank 4, so that the reading means can recognize the unique code associated with the tank 4. The mobile unit comprises a particular multi-parameter sensor, which is comparable in shape to a tube, is strong and is easy to handle.

In particular, when the user with the portable control unit 2 approaches the RFID circuit associated with the tank, the adapted receiver receives the signal emitted by the RFID circuit and the visual interface 2a of the portable control unit 2 indicates that the tank 4 is recognized by the portable control unit 2, for example by displaying a number that represents the tank 4.

As a consequence, the control unit 2 enters operational condition.

At this point, the user immerses the probe 3 inside the tank 3, for example until it reaches and rests on the bottom of the interior. The level sensor autonomously determines the zero point from which the probe 3 begins to take measurements using the sensors associated with it.

Thus, the visual interface 2 of the portable control unit 2 displays the time necessary for the probe 3 to carry out the measurements, for example by emitting a red light, and indicates when the user can raise the probe 3 again by emitting a green light.

The user will then raise the probe 3 until the visual interface 2a displays a red light, which indicates that another height level in the tank 4 has been reached in which to execute the measurements, such as, for example, an intermediate height level between the bottom of the tank 4 and the free surface of the fermenting product. When a green light is displayed again on the visual interface 2a of the portable control unit 2, the user will raise the probe 3 toward a new height level in the tank 4, which will be indicated by the reappearance of a red light.

Such operations can be performed for various different height levels of the tank 4 until reaching a region proximate to the free surface of the fermenting product.

In this manner, in a few moments, monitoring of the fermentation parameters is carried out and the data read by the probe 3 is sent, wirelessly, to the fixed control station 6, with immediate storage thereof in its data storage database.

An adapted software program, loaded in the memory means of the portable control unit 2, enables the user to view, by way of adapted graphic representations, the state of the fermentation process underway in the product contained in the tank 4, and informs the user of the need to carry out some measured interventions provided for by the work protocol, such as for example particular additions of fermentation adjuvants or enological products or the like.

The software can consist of an "expert system" which, on the basis of the data collected, makes it possible to develop consequent intervention actions.

The graphic representation of the data that is processed by the software and which can be consulted using the visual interface 2a of the portable control unit 2 can be dynamic and is capable of simply summing up the actual situation of the processes underway, highlighting only the situations in which the technologist is required to make a decision.

If the processing means of the portable control unit 2 detect a deviation of the parameters measured by the probe 3 with respect to the preset desired values specified in the work protocol, the portable control unit 2 sends a signal requesting intervention, in the form for example of an email or SMS text message, to a remote PC or a smartphone, which can constitute a second control unit 5 in the possession of the technologist.

The portable control unit 2 furthermore acquires, by way of its reception means, the data supplied by the means of measuring the heat exchanged between the heat exchanger associated with the tank 4 and the mass of fermenting product and, by way of its processing means, displays a graphic on the visual interface 2a representing the actual trend of the fermentation.

In fact, the fermenting activity of the yeasts generates heat, which determines a rise in temperature in the fermenting product.

Therefore measuring the heat exchanged between the heat exchanger associated with the tank 4 and the fermenting product makes it possible to know the existing fermenting activity, in this manner it being even possible to detect latent states of stalled fermentation, in which the temperature neither increases nor decreases and there is, therefore, no exchange of heat between the product in the tank 4 and the heat exchanger designed to control the temperature of that product.

With such solution, it is even possible therefore to act to take steps to deal with phenomena of cooling owing to scant fermentation activity, even before it can become a problem.

The apparatus according to the invention is, likewise, capable of autonomously generating the batch for the product being processed, right from its first process phase, tracking it at each transfer and at each blending with another batch. Consequently, associated with each batch is the history of the processing, which is stored at each individual step, and is consultable, with charts, statistics and even with history lay-out panels, using the fixed control station 6 or the portable control unit 2.

More specifically, with the insertion of the probe 3 in the tank 4, it is possible to monitor the transfer of a determined quantity of product to another tank, thanks to the control of the volume withdrawn, which is executed using the measurements originating from the level probe associated with the probe 3. The portable control unit 2 can, in such case, be provided with a device for the remote actuation of the pump used for the transfer of the product, which can be activated and, subsequently, deactivated by the portable control unit 2, when the desired volume of product has been transferred completely from one tank to the other.

Thanks to the RFID electronic circuits associated with the tanks in the cellar, we can identify the tank from which the product is taken and the tank into which the product is loaded.

The portable control unit 2 is thus capable of associating the transfer operation carried out with the tanks involved, thus making it possible to track the volumes of product involved in the transfer.

In practice it has been found that the invention is capable of fully achieving the set aim and objects and, in particular, it should be noted that the apparatus according to the invention makes it possible to speed up operations of monitoring fermentation processes, without high installation burdens.

The apparatus according to the invention brings a plurality of further advantages which are highlighted hereinbelow.

In particular, the apparatus according to the invention has the advantage that it makes it possible to obtain analyses on a plurality of tanks in a cellar, even more than once per day, and to obtain analyses at different height levels, determined automatically, in the tanks, without needing to extract samples.

Another advantage of the apparatus, according to the invention, is that it makes it possible to monitor the trend of fermentation precisely and directly, thanks to the ability to measure the exchange of heat that is generated during the fermentation.

The apparatus according to the invention also makes it possible to obtain fermentations by containing the daily consumption of sugars to a determined parameter, thanks to the possibility of immediate analysis of the fermentation parameters.

It should furthermore be noted that the apparatus according to the invention makes it possible to obtain fermentations that are periodically managed and are associated with mutually interpolated parameters.

Another advantage of the apparatus according to the invention is that it makes it possible to anticipate the onset of stalled fermentations, by simultaneously monitoring many parameters that are strategic to fermentation.

It should also be noted that, with little structural intervention, the apparatus according to the invention ensures total control of the batches in their processes.

It should also be noted that the apparatus according to the invention makes it possible to generate requests for intervention only if unforeseen conditions exist, requiring the targeted intervention of the technologist.

A further important advantage of the apparatus according to the invention is that it makes it possible to significantly reduce laboratory analysis costs, thus freeing up staff from extraordinary seasonal activity, as well as making it possible to optimize technical human resources, by involving them in the planning of protocols in periods leading up to the harvest season.

Another advantage of the apparatus according to the invention is that it makes it possible to organize the application protocols in a standardized and repeatable manner, enabling their methodic filing, consultation and comparison.

The apparatus according to the invention also makes it possible to achieve the total transparency of the production process, making it possible to document and thus certify all the work done that is associated with a determined production batch, in addition to making it possible to manage the partial blending of different batches, by documenting the history of each individual batch that makes up the final mass.

Another advantage of the apparatus according to the invention is that it makes it possible, automatically, to command the shutdown of the offtake pump during the blending of different batches.

All the characteristics of the invention, indicated above as advantageous, convenient or similar, may also be missing or be substituted by equivalent characteristics.

The individual characteristics set out in reference to general teachings or to specific embodiments may all be present in other embodiments or may substitute characteristics in such embodiments.

The invention, thus conceived, is susceptible of numerous modifications and variations, all of which are within the scope of the appended claims.

In practice the materials employed, provided they are compatible with the specific use, and the dimensions and shapes, may be any according to requirements.

Moreover, all the details may be substituted by other, technically equivalent elements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus for monitoring and controlling fermentation processes, comprising a detection probe (3) and a portable control unit (2) provided with means for communication with the detection probe (3), wherein the detection probe (3) can be immersed at different height levels within a fermenting product contained in a tank (4), said probe (3) having at least one sensor adapted to detect a respective parameter that is directly correlated to the fermentation process in progress in said product, said portable control unit (2) being provided with means for treating the data that originate from said probe (3); said portable control unit (2) being provided with means for recognition of said tank (4); said apparatus being **characterized by** said probe (3) further comprising at least one level sensor adapted to indicate to said portable control unit (2) the position of said probe (3) within said recognized tank (4).

2. The apparatus according to claim 1, **characterized in that** said communication means comprise a data transmission cable (3a) or are of the wireless type.

3. The apparatus according to one or more of the preceding claims, **characterized in that** said recognition means comprise at least one unique code associated with said tank (4) and means for reading said unique code which are associated with said portable control unit (2).

4. The apparatus according to one or more of the preceding claims, **characterized in that** said treatment means comprise a memory that contains a preset work protocol and processing means adapted to compare the data received from said probe (3) with said work protocol.

5. The apparatus according to claim 4, **characterized in that** said portable control unit (2) comprises at least one visual interface (2a) connected to said processing means.

6. The apparatus according to claim 5, **characterized in that** said visual interface (2a) is adapted to emit a first signal when said probe (3) reaches at least one preset level of height from the bottom of said tank (4).

7. The apparatus according to claim 6, **characterized in that** said visual interface is adapted to emit at least one second signal upon completion of the detection operations performed by said probe (3), at said at least one preset height level.

8. The apparatus according to one or more of the preceding claims, **characterized in that** said portable control unit (2) comprises wireless transceiver means for communicating information to a second control unit (5).

9. The apparatus according to claim 8, **characterized in that** said second control unit (5) comprises a PC or a smartphone.

10. The apparatus according to claims 8 or 9, **characterized in that** said second control unit comprises a fixed control station (6) provided with a data storage database.

11. The apparatus according to one or more of the preceding claims 4 to 10, **characterized in that** said processing means are adapted to send, by means of said wireless transceiver means, an intervention signal to said second control unit (5) upon detection of a variation between the data detected by said probe (3) and said work protocol by said processing means.

12. The apparatus according to one or more of the preceding claims, **characterized in that** said probe (3) comprises at least one sensor that is selected from the group comprising an oxygen sensor, a conductivity sensor, an ammonia nitrogen sensor, a turbidity sensor, a pH sensor, a sugar sensor.

13. The apparatus according to one or more of the preceding claims, **characterized in that** said portable control unit (2) is provided with means for receiving the data that arrive from means for measuring the heat exchanged between said tank (4) and a heat exchanger associated with said tank (4), said heat exchanger being designed to control the temperature of said fermenting product.

## Patentansprüche

1. Vorrichtung zur Überwachung und Kontrolle von Fermentationsprozessen, umfassend eine Detektionssonde (3) und eine tragbare Kontrolleinheit (2), die mit Mitteln zur Kommunikation mit der Detektionssonde (3) ausgestattet ist, wobei die Detektionssonde (3) in unterschiedlichen Höhenniveaus innerhalb eines Fermentationsprodukts, das in einem Behälter (4) enthalten ist, eingetaucht werden kann, welche Detektionssonde (3) mindestens einen Sensor besitzt, der geeignet ist, um einen jeweiligen Parameter zu bestimmen, welcher mit dem Fermentationsprozess, der in dem genannten Produkt stattfindet, direkt in Beziehung steht, welche tragbare Kontrolleinheit (2) mit Mitteln zur Behandlung der Daten ausgestattet ist, die von der genannten Sonde (3) stammen; welche tragbare Kontrolleinheit (2) mit Mitteln zur Erkennung des genannten Behälters (4) ausgestattet ist; wobei die Vorrichtung **dadurch gekennzeichnet ist, dass**
die genannte Sonde (3) ferner mindestens einen Niveausensor umfasst, welcher geeignet ist, um der genannten tragbaren Kontrolleinheit (2) die Position der genannten Sonde (3) innerhalb des genannten erkannten Behälters (4) anzuzeigen.

2. Vorrichtung nach Anspruch 1, **dadurch gekennzeichnet, dass**
die Mittel zur Kommunikation ein Datenübertragungskabel (3a) umfassen oder vom drahtlosen Typ sind.

3. Vorrichtung nach einem oder mehreren der vorstehenden Ansprüche, **dadurch gekennzeichnet, dass** die genannten Erkennungsmittel mindestens einen eindeutigen Code umfassen, welcher mit dem genannten Behälter (4) assoziiert ist, und Mittel zum Lesen des genannten eindeutigen Codes, die mit der genannten tragbaren Kontrolleinheit (2) assoziiert sind.

4. Vorrichtung nach einem oder mehreren der vorstehenden Ansprüche, **dadurch gekennzeichnet, dass** die genannten Behandlungsmittel einen Speicher umfassen, welcher ein vorbestimmtes Arbeitsprotokoll enthält, und Verarbeitungsmittel, die geeignet sind, um die von der genannten Sonde (3) erhaltenen Daten mit dem genannten Arbeitsprotokoll zu vergleichen.

5. Vorrichtung nach Anspruch 4, **dadurch gekennzeichnet, dass**
die genannte tragbare Kontrolleinheit (2) mindestens eine optische Schnittstelle (2a) umfasst, welche mit den genannten Verarbeitungsmitteln verbunden ist.

6. Vorrichtung nach Anspruch 5, **dadurch gekennzeichnet, dass**
die genannte optische Schnittstelle (2a) geeignet ist, um ein erstes Signal auszusenden, wenn die genannte Sonde (3) mindestens ein vorbestimmtes Höheniveau vom Boden des genannten Behälters (4) erreicht.

7. Vorrichtung nach Anspruch 6, **dadurch gekennzeichnet, dass**
die optische Schnittstelle geeignet ist, um bei Beendigung der Detektionsschritte, welche von der genannten Sonde (3) durchgeführt werden, mindestens ein zweites Signal in dem genannten mindestens einen vorbestimmten Höhenniveau auszusenden.

8. Vorrichtung nach einem oder mehreren der vorstehenden Ansprüche, **dadurch gekennzeichnet, dass** die genannte tragbare Kontrolleinheit (2) drahtlose Sendemittel zur Kommunikation von Information an eine zweite Kontrolleinheit (5) umfasst.

9. Vorrichtung nach Anspruch 8, **dadurch gekennzeichnet, dass**
die genannte zweite Kontrolleinheit (5) einen PC oder ein Smartphone umfasst.

10. Vorrichtung nach Anspruch 8 oder 9, **dadurch gekennzeichnet, dass** die genannte zweite Kontrolleinheit eine stationäre Kontrollstation (6) umfasst, welche mit einer Datenspeicher-Datenbank ausgestattet ist.

11. Vorrichtung nach einem oder mehreren der vorstehenden Ansprüche 4 bis 10, **dadurch gekennzeichnet, dass** die genannten Verarbeitungsmittel geeignet sind, um bei Feststellen eines Unterschieds zwischen den Daten, welche von der genannten Sonde (3) detektiert werden, und dem genannten Arbeitsprotokoll durch die genannten Verarbeitungsmittel mit Hilfe der genannten drahtlosen Sendemittel ein Interventionssignal an die genannte zweite Kontrolleinheit (5) zu senden.

12. Vorrichtung nach einem oder mehreren der vorstehenden Ansprüche, **dadurch gekennzeichnet, dass** die genannte Sonde (3) mindestens einen Sensor umfasst, welcher von der Gruppe, umfassend einen Sauerstoffsensor, einen Sensor für die Leitfähigkeit, einen Sensor für Ammoniakstickstoff, einen Trübungssensor, einen pH-Sensor, einen Zuckersensor, ausgewählt ist.

13. Vorrichtung nach einem oder mehreren der vorstehenden Ansprüche, **dadurch gekennzeichnet, dass** die genannte tragbare Kontrolleinheit (2) mit Mitteln ausgestattet ist, um die Daten zu erhalten, welche von Mitteln zur Messung der Wärme ankommen, die zwischen dem genannten Behälter (4) und einem Wärmetauschen ausgetauscht wird, der mit dem genannten Behälter (4) assoziiert ist, welcher Wärmetauscher bestimmt ist, um die Temperatur des genannten fermentierenden Produkts zu kontrollieren.

## Revendications

1. Dispositif pour contrôler et commander des processus de fermentation, comprenant une sonde de détection (3) et une unité de commande portable (2) pourvue de moyens pour communication avec la sonde de détection (3), dans lequel la sonde de détection (3) peut être immergée à différents niveaux de hauteur dans un produit fermentant contenu dans un réservoir (4), ladite sonde (3) comportant au moins un capteur adapté pour détecter un paramètre respectif qui est directement corrélé au processus de fermentation en progrès dans ledit produit, ladite unité de commande portable (2) étant pourvue de moyens pour traiter les données qui proviennent de ladite sonde (3) ; ladite unité de commande portable (2) étant pourvue de moyens pour reconnaissance dudit réservoir (4) ; ledit dispositif étant **caractérisé par**
ladite sonde (3) comprenant en outre au moins un capteur de niveau adapté pour indiquer à ladite unité de commande portable (2) la position de ladite sonde (3) dans ledit réservoir (4) reconnu.

2. Dispositif selon la revendication 1, **caractérisé en ce que** lesdits moyens de communication comprennent un câble de transmission de données (3a) ou sont du type sans fil.

3. Dispositif selon une ou plus des revendications précédentes, **caractérisé en ce que** lesdits moyens de reconnaissance comprennent au moins un code unique associé audit réservoir (4) et des moyens pour lire ledit code unique qui sont associés à ladite unité de commande portable (2).

4. Dispositif selon une ou plus des revendications précédentes, **caractérisé en ce que** lesdits moyens de traitement comprennent une mémoire qui contient un protocole de travail préétabli et des moyens de traitement adaptés pour comparer les données reçues depuis ladite sonde (3) avec ledit protocole de travail.

5. Dispositif selon la revendication 4, **caractérisé en ce que**
ladite unité de commande portable (2) comprend au moins une interface visuelle (2a) connectée auxdits moyens de traitement.

6. Dispositif selon la revendication 5, **caractérisé en ce que**
ladite interface visuelle (2a) est adaptée pour émettre un premier signal quand ladite sonde (3) atteint au moins un niveau préétabli de hauteur relativement au fond dudit réservoir (4).

7. Dispositif selon la revendication 6, **caractérisé en ce que**
ladite interface visuelle est adaptée pour émettre au moins un second signal lors de l'accomplissement des opérations de détection réalisées par ladite sonde (3), audit au moins un niveau de hauteur préétabli.

8. Dispositif selon une ou plus des revendications précédentes, **caractérisé en ce que** ladite unité de commande portable (2) comprend des moyens formant émetteur-récepteur sans fils pour communiquer des informations à une seconde unité de commande (5).

9. Dispositif selon la revendication 8, **caractérisé en ce que**
ladite seconde unité de commande (5) comprend un PC ou un Smartphone.

10. Dispositif selon les revendications 8 ou 9, **caractérisé en ce que** ladite seconde unité de commande comprend une station de commande fixe (6) pourvue d'une base de données de stockage de données.

11. Dispositif selon une ou plus des revendications précédentes 4 à 10, **caractérisé en ce que** lesdits moyens de traitement sont adaptés pour envoyer, au moyen desdits moyens formant émetteur-récepteur sans fil, un signal d'intervention à ladite seconde unité de commande (5) lors de la détection d'une variation entre les données détectées par ladite sonde (3) et ledit protocole de travail par lesdits moyens de traitement.

12. Dispositif selon une ou plus des revendications précédentes, **caractérisé en ce que** ladite sonde (3) comprend au moins un capteur qui est sélectionné parmi le groupe comprenant un capteur d'oxygène, un capteur de conductivité, un capteur d'azote ammoniacal, un capteur de turbidité, un capteur de PH, un capteur de sucre.

13. Dispositif selon une ou plus des revendications précédentes, **caractérisé en ce que** ladite unité de commande portable (2) est pourvue de moyens pour recevoir les données qui arrivent depuis des moyens pour mesurer la chaleur échangée entre ledit réservoir (4) et un échangeur de chaleur associé audit réservoir (4), ledit échangeur de chaleur étant conçu pour commander la température dudit produit fermentant.
